# EUROPEAN PATENT APPLICATION

(11) **EP 2 363 161 A1**
(43) Date of publication of application: **07.09.2011**
(21) Application number: 11394004.3
(22) Date of filing: 28.02.2011
(51) Int. Cl.: A61M 11/04, A61M 15/06, A24F 47/00

(54) **A vaporising device**

(30) Priority: 01.03.2010 IE 20100112
(71) Applicant: OGLESBY & BUTLER, RESEARCH & DEVELOPMENT LIMITED, Carlow (IE)
(72) Inventor: Oglesby, Alfred Peter, Carlingford County Louth (IE); Townsend, John, Carlow (IE)
(74) Representative: Gorman, Francis Fergus

(57) **Abstract**

A vaporising device (1) for vaporising herbal matter comprises a body member 7, within which a vaporising chamber (9) is formed for the herbal matter (2). A heat conducting spigot (43) extends into the vaporising chamber (9). A heat transfer element (45) comprising a carrier ring (46) and three equi-spaced apart heat transfer spigots (47) extending from the carrier ring (46) is removeably engageable in the vaporising chamber (9) for transferring heat directly into the herbal matter. A gas catalytic combustion element (12) located in a combustion chamber (10) formed in the body member (7) converts fuel gas to heat which is transferred through the body member (7), the heat conducting spigot (43) and the heat transfer spigots (47) of the heat transfer element (45) to the herbal matter in the vaporising chamber (9). The heat transfer element (45) is removable and replaceable by hand in the vaporising chamber (9) for cleaning thereof and for permitting cleaning of the vaporising chamber (9).

## Description

The present invention relates to a vaporising device for vaporising vaporisable matter to produce an aerosol for inhaling, and in particular, though not limited to a device for vaporising flavour constituents, medicinal and medical constituents and psychoactive constituents from such matter. Such vaporisable matter may be, for example, tobacco, mullein, passionflower, cloves, yohimbe, mint, tea, eucalyptus, camomile and other such herb and plant matter, as well as medicinal compounds, although the invention is not limited to a vaporising device for vaporising such matter. The invention also relates to a vaporising device for vaporising medicinal compounds and other matter to form an aerosol for inhaling for rapid absorption into the bloodstream. Further the invention provides a heat transfer element for use in the vaporising device.

Vaporising devices for vaporising vaporisable constituents of vaporisable matter are known. Examples of such vaporising devices are disclosed in Published PCT Patent Applications Specifications Nos. WO 2006/082571, WO 2008/029381 and WO 2009/027959.

In general, such vaporising devices comprise a vaporising chamber for the vaporisable matter, and a heating means for heating the vaporisable matter in the vaporising chamber in order to vaporise vaporisable constituents in the vaporisable matter. The heating means may be electrically powered or gas powered. A mouthpiece which communicates with the vaporising chamber is provided to facilitate drawing of an aerosol which comprises vapours from the vaporisable constituents of the vaporisable matter from the vaporising chamber. By drawing on the mouthpiece, air is drawn into the vaporising chamber, and vapours from the vaporisable constituents are entrained in the air as it is drawn through the vaporising chamber to form the aerosol. The aerosol is then drawn from the vaporising chamber through the mouthpiece and inhaled.

The heating means maintains the vaporisable matter in the vaporising chamber substantially at the vaporising temperature of the vaporisable constituent or constituents of the vaporisable matter. In the case of herbal matter, depending on the herbal matter, the vaporising temperature may typically lie in the range of 120°C to 230°C.

However, in order for the vaporisable constituents to be vaporised, it is essential that the temperature of the vaporisable matter in the vaporising chamber is maintained at the vaporising temperature of the relevant vaporisable constituent or constituents. This requires that the variation in the temperature of the temperature profile within the vaporising chamber should be minimal. It is also desirable that the vaporisable matter is raised to the vaporising temperature as quickly as possible from start-up of the vaporising device. Furthermore, it is desirable that the vaporising chamber should be easily cleaned.

The present invention is directed towards providing a vaporising device which addresses these issues, and also the invention is directed towards providing a heat transfer element for a vaporising device which likewise addresses these issues.

According to the invention there is provided a vaporising device for vaporising a vaporisable constituent of vaporisable matter, the vaporising device comprising a vaporising chamber for the vaporisable matter, a heating means a heat transfer means extending into the vaporising chamber adapted to contact the vaporisable matter therein for transferring heat to the vaporisable matter, the heat transfer means being adapted to be removeably engageable in the vaporising chamber.

In one embodiment of the invention the vaporising chamber is formed within a body member, and the heating means is adapted to heat the body member. Preferably, the body member is of heat conductive material.

In one embodiment of the invention the heat transfer means is adapted to engage the body member for transferring heat therefrom to the vaporisable matter. Advantageously, the heat transfer means is adapted to engage the body member with heat conducting engagement for conducting heat from the body member. Preferably, the heat transfer means is adapted for engaging the body member within the vaporising chamber.

In another embodiment of the invention the heat transfer means comprises a heat transfer element comprising a carrier means, and at least one heat transfer spigot extending from the carrier means, the carrier means being adapted to engage the body member. Preferably, a plurality of spaced apart heat transfer spigots extend from the carrier means. Advantageously, each heat transfer spigot terminates in a distal tapering portion. Preferably, the distal tapering portion of each heat transfer spigot terminates in a puncturing point.

In one embodiment of the invention the heat transfer spigots are spaced apart circumferentially around the carrier means.

In a further embodiment of the invention the carrier means defines a heat transfer surface adapted to abut a corresponding heat transfer surface of the body member within the vaporising chamber for heat transfer therebetween. Preferably, the heat transfer surface of the body member defines the heat transfer surface of the carrier means.

In one embodiment of the invention the carrier means defines an opening extending therethrough for accommodating a fixed heat conducting spigot extending from the body member into the vaporising chamber.

In another embodiment of the invention the heat conducting spigot is rigidly connected to the body member.

In one embodiment of the invention the carrier means comprises a carrier plate member.

In another embodiment of the invention the carrier means comprises a carrier ring.

In a further embodiment of the invention the body member forms a socket element having a base, and a side wall extending from and around the base and defining with the base the vaporising chamber. Preferably, the heat transfer surface of the body member is formed in the base of the socket element. Advantageously, an annular groove is formed extending around and into the base of the socket element for accommodating the carrier means of the heat transfer element, and the heat transfer surface of the body member is formed by the surface of the annular groove.

In another embodiment of the invention a hollow plug element is releasably engageable in the socket element, the plug element defining a hollow interior region for the vaporisable matter and an open mouth to the hollow interior region, the plug element being engageable in the socket element for defining with the socket element the vaporising chamber. Advantageously, the plug element engages the socket element with heat conducting engagement. Preferably, the plug element is adapted to engage the socket element so that when the plug element is engaged in the socket element, the heat transfer means extends into the hollow interior region of the plug element to contact the vaporisable matter therein. Advantageously, the plug element co-operates with the socket element to define an air accommodating passageway into the vaporising chamber between the plug element and the socket element.

In another embodiment of the invention the air accommodating passageway is defined between the plug element and the side wall of the socket element. Preferably, the air accommodating passageway is located relative to the vaporising chamber so that air flows through the air accommodating passageway in a direction opposite to the direction of flow of air through the vaporising chamber. Advantageously, the air accommodating passageway defined between the plug element and the socket element is adapted to pre-heat air to the vaporising chamber.

In one embodiment of the invention a communicating means extends from the vaporising chamber for accommodating an aerosol therefrom, the communicating means terminating in a mouthpiece. Preferably, the communicating means extends from the plug element. Advantageously, the communicating means comprises a communicating tube extending from the vaporising chamber to the mouthpiece.

In another embodiment of the invention the heating means comprises a gas powered heating means. Preferably, the heating means comprises a combustion chamber. Advantageously, the heating means comprises a gas catalytic combustion element which is located in the combustion chamber. Ideally, the combustion chamber is formed in the body member.

In another embodiment of the invention a means is provided for delivering fuel gas to the heating means.

Alternatively, the heating means comprises an electrically powered heating means. Preferably, the heating means comprises at least one electrically powered heating element located in the body member.

In one embodiment of the invention the heat transfer means is adapted to be manually removeable and insertable in the vaporising chamber.

Additionally, the invention provides a heat transfer element for the vaporising device according to the invention.

The invention also provides a heat transfer element adapted for use in a vaporising device of the type comprising a vaporising chamber for vaporisable matter, the heat transfer element being adapted to contact vaporisable matter in the vaporising chamber for transferring heat to the vaporisable matter, and being adapted for removeable engagement in the vaporising chamber.

In one embodiment of the invention the heat transfer element is adapted to engage a body member within which the vaporising chamber is formed. Preferably, the heat transfer element is adapted to engage the body member with heat conducting engagement.

In one embodiment of the invention the heat transfer element comprises a carrier means, and at least one heat transfer spigot extending from the carrier means. Preferably, a plurality of spaced apart heat transfer spigots extend from the carrier means. Advantageously, the heat transfer spigots are equi-spaced apart circumferentially around the carrier means.

In one embodiment of the invention the carrier means defines a heat transfer surface adapted to abut a corresponding heat transfer surface of the body member within the vaporising chamber for heat transfer therebetween. Preferably, the heat transfer surface of the carrier means defines the heat transfer surface of the body member.

In another embodiment of the invention the carrier means defines an opening extending therethrough for accommodating a fixed heat conducting spigot extending from the body member into the vaporising chamber.

In one embodiment of the invention the carrier means comprises a carrier plate member.

In another embodiment of the invention the carrier means comprises a carrier ring.

Preferably, the carrier means is adapted to engage an annular groove formed in the body member.

In another embodiment of the invention the heat transfer element is adapted to be manually removeable and insertable in the vaporising chamber.

The advantages of the invention are many. The vaporising device according to the invention is particularly efficient, and brings the temperature of the vaporisable matter relatively quickly to the vaporising temperature of the vaporisable constituent or constituents of the vaporisable matter to be vaporised. Furthermore, the temperature of the vaporisable matter is maintained substantially constant at the relevant vaporising temperature during use of the vaporising device. Additionally, the heat transfer spigots of the heat transfer means maintain the temperature of the temperature profile within the vaporising chamber substantially constant both across and along the vaporising chamber. These advantages are largely achieved by the provision of the heat transfer element which significantly increases heat transfer into the vaporisable matter in the vaporising chamber. The provision of the heat transfer spigots extending from the carrier means into the vaporising chamber which contact the vaporisable matter with heat transfer contact for transferring heat into the vaporisable matter in the vaporising chamber provide a relatively even distribution of heat throughout the vaporisable matter in the vaporising chamber, and thereby maintain a relatively even constant temperature throughout the vaporising chamber. The provision of the heat transfer element as being removeably engageable in the vaporising chamber, and in particular being manually removeable, has the important advantage that the heat transfer element can be readily easily lifted out of the vaporising chamber by hand without the need for any tools. Once the heat transfer element has been removed from the vaporising chamber, cleaning of the heat transfer element can be readily easily carried out, and of particular importance, thorough cleaning of the vaporising chamber can also be readily easily carried out. Without the heat transfer element being removable from the vaporising chamber, cleaning of the vaporising chamber would otherwise be difficult. On completion of cleaning, the heat transfer element can then be returned to and placed by hand in the vaporising chamber without the need for any tools. Additionally, the fact that the heat transfer element is removeably engageable with the vaporising chamber also allows the heat transfer element to be removed from the vaporising chamber in the event that the heat required to vaporise the vaporisable constituent or constituents of the vaporisable matter to be vaporised in the vaporising chamber is such that sufficient heat can be transferred to the vaporisable matter without the need for the heat transfer element through the heat central heat conductive spigot.

The provision of the heat transfer surface of the body member within the vaporising chamber defining the heat transfer surface of the carrier means of the heat transfer means provides the advantage that the area of contact between the carrier means and the body member is maximised, thereby maximising the efficiency of heat transfer from the body member to the heat transfer means.

The advantages of the heat transfer element are similar to those discussed with respect to the vaporising device.

The invention will be more clearly understood from the following description of some preferred embodiments thereof, which are given by way of example only, with reference to the accompanying drawings, in which:
Fig. 1 is a perspective view of a vaporising device according to the invention for vaporising herbal matter,
Fig. 2 is a side elevational view of the vaporising device of Fig. 1 with a portion of the vaporising device removed,
Fig. 3 is a transverse cross-sectional side elevational view of a portion of the vaporising device of Fig. 1,
Fig. 4 is a side elevational view of a portion of the vaporising device of Fig. 1,
Fig. 5 is a perspective view of a detail of the vaporising device of Fig. 1,
Fig. 6 is a transverse cross-sectional side elevational view of a detail of Fig. 5 of the vaporising device of Fig. 1 on the line VI-VI of Fig. 4,
Fig. 7 is a perspective view of another detail of the vaporising device of Fig. 1,
Fig. 8 is a side elevational view of the detail of Fig. 7 of the vaporising device of Fig. 1,
Fig. 9 is an end elevational view of the detail of Fig. 7 of the vaporising device of Fig. 1,
Fig. 10 is a perspective view of another detail of the vaporising device of Fig. 1,
Fig. 11 is another perspective view of the detail of Fig. 10 of the vaporising device of Fig. 1,
Fig. 12 is a side elevational view of the detail of Fig. 10 of the vaporising device of Fig. 1,
Fig. 13 is an end elevational view of the detail of Fig. 10 of the vaporising device of Fig. 1,
Fig. 14 is a transverse cross-sectional plan view of the detail of Fig. 10 of the vaporising device of Fig. 1 on the line XIV-XIV of Fig. 12,
Fig. 15 is a schematic representation of another detail of the vaporising device of Fig. 1,
Fig. 16 illustrates graphs of plots of temperature against time of the temperature of parts of the vaporising device of Fig. 1 during operation thereof,
Fig. 17 is a perspective view of a heat transfer element according to another embodiment of the invention which is suitable for use in a vaporising device also according to another embodiment of the invention,
Fig. 18 is a side elevational view of the heat transfer element of Fig. 17,
Fig. 19 is an end view of the heat transfer element of Fig. 17 from one end thereof,
Fig. 20 is an end view of the heat transfer element of Fig. 17 from the other end thereof to that of Fig. 19,
Fig. 21 is a transverse cross-sectional side elevational view of a portion of a vaporising device also according to the invention illustrating the heat transfer element of Fig. 17 in the vaporising device,
Fig. 22 is a perspective view of a heat transfer element according to another embodiment of the invention which is suitable for use in a vaporising device,
Fig. 23 is a side elevational view of the heat transfer element of Fig. 22, and
Fig. 24 is an end elevational view of the heat transfer element of Fig. 22.

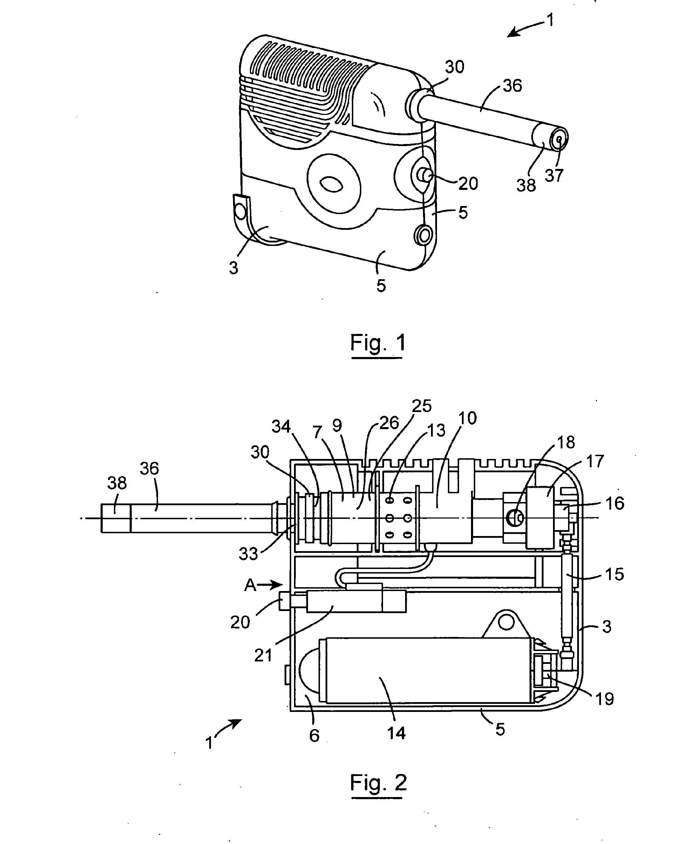

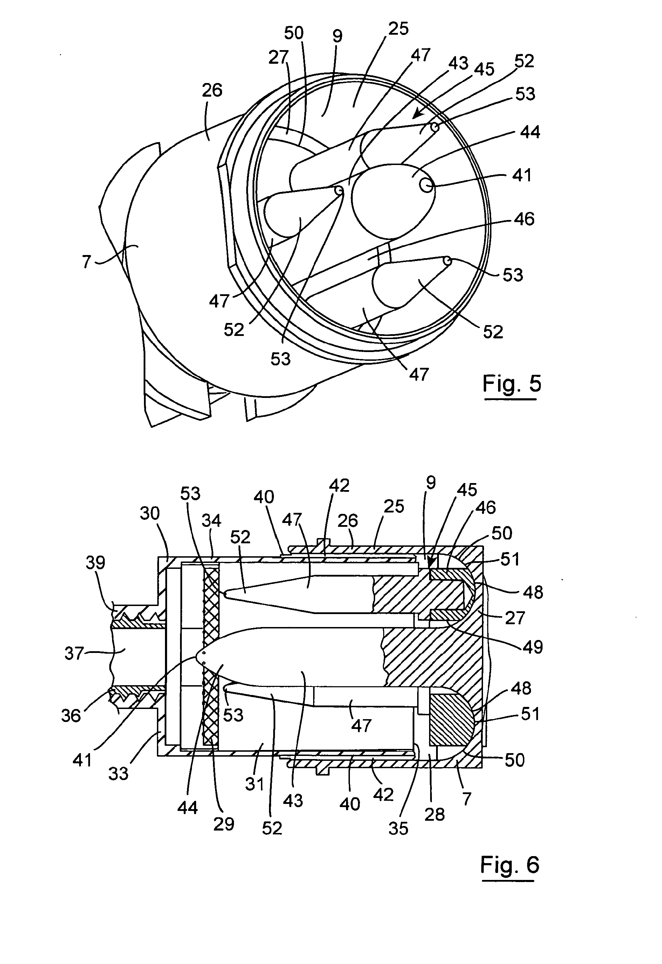

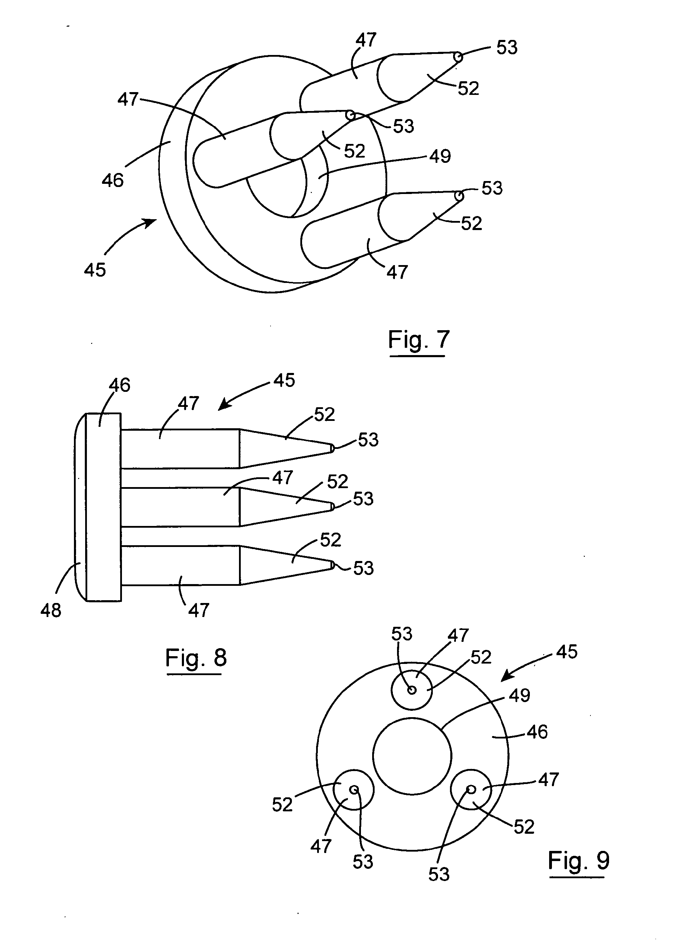

Referring to the drawings, and initially to Figs. 1 to 16, there is illustrated a vaporising device according to the invention, indicated generally by the reference numeral 1, for vaporising vaporisable matter, which in this embodiment of the invention is herbal matter 2, for example, tobacco, mullein, passionflower, cloves, yohimbe, mint, tea, eucalyptus, camomile and other such herb and plant matter. Where the herbal matter 2 is tobacco, the tobacco may be in flake, strip, particulate or any other suitable form, or a mixture of two or more such forms. The vaporising device comprises a two-part casing 3 formed by a pair of shells 5, which when secured together define a hollow interior region 6.

An elongated body member 7 of generally circular transverse cross-section of heat conductive material, which in this case is brass, forms a vaporising chamber 9 for the herbal matter 2 and a combustion chamber 10. A heating means, namely, a gas catalytic combustion element 12 is located in the combustion chamber 10 for converting fuel gas to heat. Exhaust ports 13 extending through the body member 7 from the combustion chamber 10 accommodate exhaust gases from the combustion chamber 10. Heat from the combustion chamber 10, which is generated by the gas catalytic combustion element 12 is conducted through the body member 7 to the herbal matter 2 in the vaporising chamber 9 for heating thereof as will be described below. Fuel gas in a rechargeable fuel gas reservoir 14 located in the hollow interior region 6 of the casing 3 is delivered to the gas catalytic combustion element 12 in the combustion chamber 10 through a fuel supply pipe 15, a safety cut-out valve 16, a thermostatically controlled valve 17 and a venturi mixer 18 within which the fuel gas is mixed with air for delivery into the combustion chamber 10. An on/off valve 19 located in an outlet (not shown) from the fuel gas reservoir 14 controls the supply of fuel gas to the safety cut-out valve 16. A button actuator 20 extending through the casing 3 is connected to the on/off valve 19 by a linkage mechanism (not shown) for operating the valve 19 between an isolating state and a communicating state.

A piezoelectric igniter 21 is located in the hollow interior region 6 of the casing 3 to develop a high voltage which is applied to an electrode 22 which extends into the combustion chamber 10 to cause a spark to arc from the electrode 22 to the body member 7 within the combustion chamber 10. The spark initially ignites the fuel gas/air mixture from the venturi mixer 18 to burn in a flame for in turn raising the gas catalytic combustion element 12 to its ignition temperature. On the gas catalytic combustion element 12 reaching its ignition temperature, the gas catalytic combustion element 12 commences to convert fuel gas to heat catalytically, thereby starving the flame of fuel gas, which is then extinguished. The button actuator 20 is coupled to the piezoelectric igniter 21 so that as the button actuator 20 is depressed in the direction of the arrow A into the casing 3 to operate the on/off valve 19 into the communicating state, the piezoelectric igniter 21 is operated to apply the high voltage to the electrode 22. The button actuator 20 is coupled to the on/off valve 19 by the linkage mechanism (not shown) and is coupled to the piezoelectric igniter 21 so that the operation of the on/off valve 19 and piezoelectric igniter 21 is timed in order that sufficient fuel gas is in the combustion chamber 10 when the spark is produced in the combustion chamber 10 to commence ignition of the fuel gas to burn in a flame in the combustion chamber 10.

A vaporising device which comprises a combustion chamber, a gas catalytic combustion element, a fuel gas reservoir, a safety cut-out valve, a thermostatically controlled valve, a venturi mixer and an igniter and an isolating valve similar to those components of the vaporising device 1 is disclosed in Published PCT Patent Application Specification No. WO 2006/082571, and further description of this aspect of the vaporising device 1 should not be required.

The vaporising chamber 9 is formed in the body member 7 by a socket element 25 of circular transverse cross-section. The socket element 25 is formed by a cylindrical side wall 26 of the body member 7 extending from and around a base wall 27 also of the body member 7 which extends transversely within the body member 7. The base wall 27 and the side wall 26 define a cylindrical hollow interior region 28. A hollow plug element 30 which defines a cylindrical hollow interior region 31 is engageable in the hollow interior region 28 of the socket element 25 to form with the socket element 25 the vaporising chamber 9. The plug element 30 comprises a base wall 33 and a side wall 34 extending around and from the base wall 33, and defining with the base wall 33 the hollow interior region 31. The side wall 34 defines an open mouth 35 to the hollow interior region 31 for communicating the hollow interior region 31 of the plug element 30 with the socket element 25.

A communicating means comprising a communicating tube 36 having a communicating bore 37 extending therethrough extends from an outlet port 39 in the base wall 33 of the plug element 30. The communicating bore 37 of the communicating tube 36 communicates with the vaporising chamber 9 through the inlet port 39 for accommodating an aerosol of the vaporisable constituents of the herbal matter 2 from the vaporising chamber 9. The communicating tube 36 terminates in a mouthpiece 38 for facilitating drawing of the aerosol from the vaporising chamber 9 for inhaling thereof. A condenser 32 provided in the communicating bore 37 of the communicating tube 36 for condensing undesirable vapours from the aerosol as the aerosol is being drawn from the vaporising chamber 9 through the communicating tube 36. A gauze panel 29 located in the hollow interior region 31 of the plug element 30 adjacent to but slightly spaced apart from the base wall 33 of the plug element 30 retains the herbal matter 2 in the vaporising chamber 9, and prevents any particles of the herbal matter 2 being drawn with the aerosol from the vaporising chamber 9. The spacing of the gauze panel 29 from the base wall 33 of the plug element 30 accommodates the aerosol from the gauze panel 29 to the outlet port 39 in the base wall 33.

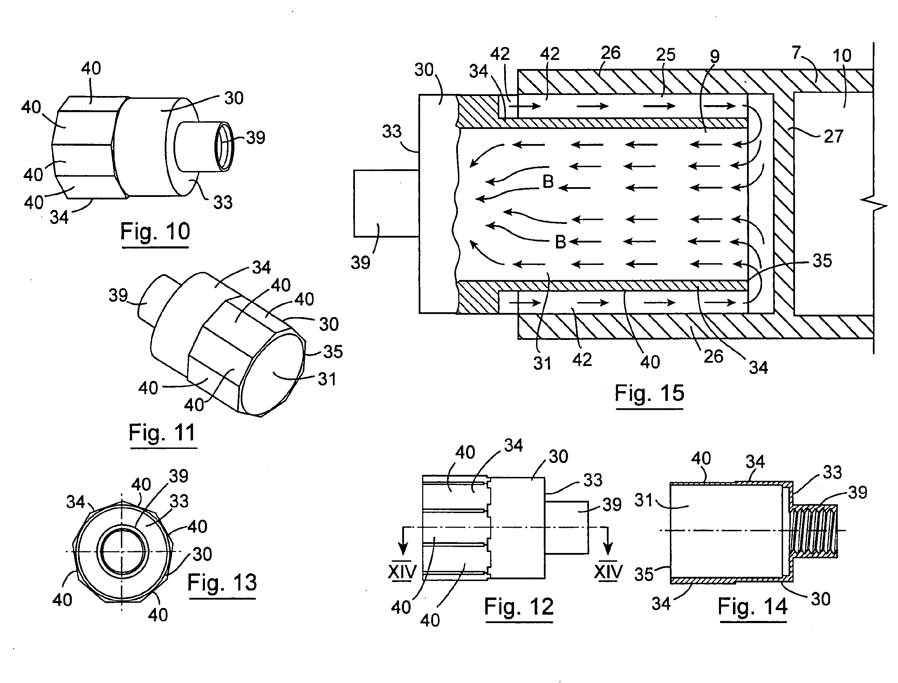

In this embodiment of the invention the outer surface of the side wall 34 of the plug element 30 is provided with flats 40 so that when the plug element 30 is engaged in the hollow interior region 28 of the socket element 25, the flats 40 of the side wall 34 are spaced apart from the cylindrical side wall 26 of the socket element 25 in order to form respective air accommodating passageways 42 for accommodating air into the vaporising chamber 9. Drawing the air into the vaporising chamber 9 through the passageways 42 formed between the plug element 30 and the socket element 25 results in pre-heating of the air as it is being drawn through the passageways 42. The air being drawn into the vaporising chamber 9 through the passageways 42 flows through the passageways 42 in the opposite direction to the direction through which the air flows through the vaporising chamber 9. A schematic view of the vaporising chamber 9 in Fig. 15 illustrates the air flow through the passageways 42 and through the vaporising chamber 9. The air flow is indicated by the arrows B.

A fixed heat conducting spigot 43 which is integrally formed with the body member 7 from the same material of the body member 7 by machining extends rigidly from the base wall 27 of the socket element 25 into the hollow interior region 28 of the socket element 25 for directly contacting the herbal matter 2 in the vaporising chamber 9, and for transferring heat from the body member 7 into the vaporisable matter 2 in the vaporising chamber 9. The fixed heat conducting spigot 43 is of circular transverse cross-section and terminates in a tapered distal end portion 44 which forms a puncturing point 41 for penetrating the herbal matter 2 in the hollow interior region 31 of the plug element 30, and also for puncturing a sachet in the event that the herbal matter is placed in the hollow interior region 31 of the plug element 30 in a sachet.

A heat transfer means for transferring heat from the body member 7 into the herbal matter 2 in the vaporising chamber 9 comprises a heat transfer element also according to the invention and indicated generally by the reference numeral 45. The heat transfer element 45 is removeably engageable in the hollow interior region 28 of the socket element 25, and is removable and replaceable in the hollow interior region 28 by hand. The heat transfer element 45 comprises a carrier means, namely, a carrier member, which in this embodiment of the invention comprises a carrier ring 46 of heat conducting material, namely, brass, stainless steel or aluminium. Three heat transfer spigots 47 of similar heat conducting material to that of the carrier ring 46 extend from the carrier ring 46 to contact the herbal matter 2 in the vaporising chamber 9 for transferring heat to the herbal matter 2. The heat transfer spigots 47 are equi-spaced apart circumferentially around the carrier ring 46 at 120° intervals.

The carrier ring 46 defines a central opening 49 for accommodating the heat conducting spigot 43 therethrough, and is partly of rectangular transverse cross-section. A heat transfer surface formed by an end face 48 of arcuate cross-section of the carrier ring 46 releasably abuts a corresponding heat transfer surface of the body member 7, which is formed by an arcuate base surface 51 of an annular groove 50 extending into the base wall 27 of the socket element 25 and extending around the fixed heat conducting spigot 43. The radius of the end face 48 of the carrier ring 46 is substantially similar to the radius of the arcuate base surface 51 of the groove 50, so that the carrier ring 46 releasably engages the groove 50 with heat conducting engagement with the end face 48 abutting the base surface 51 of the annular groove 50 for efficiently transferring heat from the base wall 27 of the socket element 25 into the carrier ring 46, and in turn through the heat transfer spigots 47 from which the heat is transferred directly into the herbal matter 2 in the vaporising chamber 9.

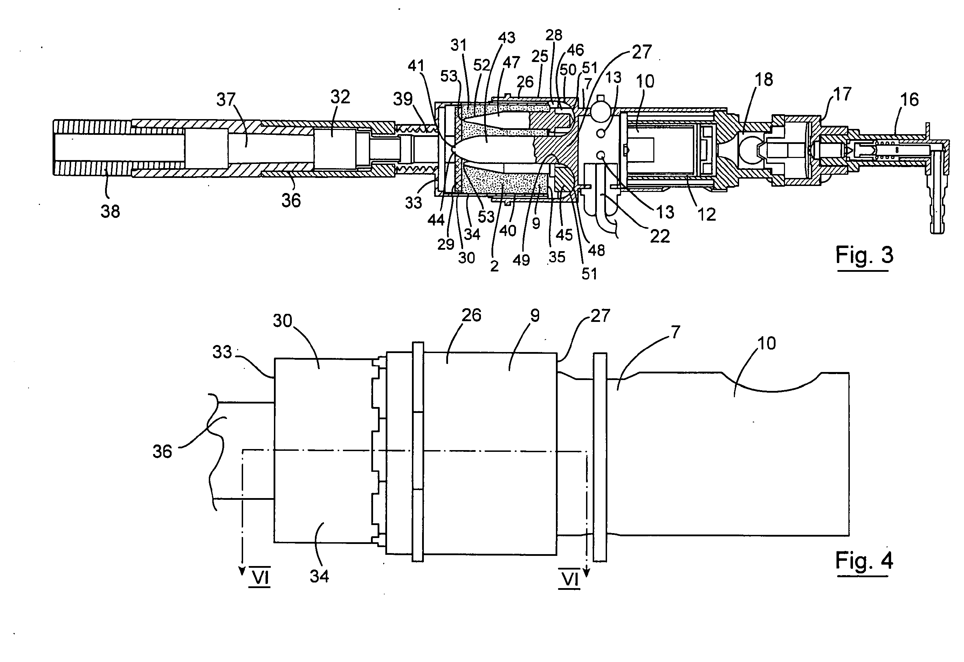

The diameter of the carrier ring 46 and the positioning of the heat transfer spigots 47 on the carrier ring 46 is such that when the carrier ring 46 is engaged in the groove 50, the heat transfer spigots 47 extend into the hollow interior region 31 of the plug element 30 and are relatively close to but slightly spaced apart from the side wall 34 of the plug element 30, so that the heat transfer spigots 47 engage the herbal matter 2 in the hollow interior region 31 of the plug element 30 with the herbal matter 2 completely surrounding the heat transfer spigots 47. This can most clearly be seen in Figs. 3, 5 and 6.

The heat transfer spigots 47 terminate in tapering distal end portions 52 which form respective puncturing points 53 for penetrating the herbal matter 2 in the hollow interior region 31 of the plug element 30, and for puncturing a sachet, in the event of the herbal matter being placed in the hollow interior region 31 of the plug element 30 in a sachet.

By virtue of the fact that the heat transfer element 45 is removeably engageable in the hollow interior region 28 of the socket element 25, the heat transfer element can readily easily be removed and replaced for cleaning thereof, and for permitting cleaning of the hollow interior region 28 of the socket element 25.

In use, with the heat transfer element 45 located in the socket element 25 and with the carrier ring 46 engaging the groove 50 in the base wall 27 of the socket element 25, herbal matter to be vaporised is placed in the hollow interior region 31 of the plug element 30. The plug element 30 is then engaged in the hollow interior region 28 of the socket element 25. As the plug element 30 is being engaged in the socket element 25, the heat conducting spigot 43 and the heat transfer spigots 47 extend into the hollow interior region 31 of the plug element 30, and penetrate into the herbal matter 2, with the herbal matter 2 surrounding the heat conducting spigot 43 and the heat transfer spigots 47. If the herbal matter is placed in the hollow interior region 31 of the plug element 30 in a sachet, the puncturing points 53 of the heat transfer spigots 47 and the puncturing point 41 of the heat conducting spigot 43 puncture the sachet, so that the heat transfer spigots 47 and the heat conducting spigot 43 extend into and penetrate the herbal matter in the sachet.

With the plug element 30 engaged in the socket element 25, the button actuator 20 is depressed into the casing 3 in the direction of the arrow A in order to operate the on/off valve 24 into the communicating state, so that fuel gas is supplied from the fuel gas reservoir 14 to the gas catalytic combustion element 12 in the combustion chamber 10, and to operate the piezoelectric igniter 21 to apply a high voltage on the electrode 22 to ignite the fuel gas/air mixture in the combustion chamber 10 to burn with a flame. The flame burning in the combustion chamber 10 raises the temperature of the gas catalytic combustion element 12 to its ignition temperature, thus resulting in the gas catalytic combustion element 12 converting the fuel gas/air mixture to heat by catalytic action. As the catalytic combustion element 12 is fully brought up to its ignition temperature, the flame is starved of fuel gas and is thus extinguished. Thereafter, the gas catalytic combustion element 12 continues to convert the fuel gas to heat by catalytic reaction.

The temperature of the body member 7, and in turn the temperature of the vaporising chamber 9 and the herbal matter 2 therein is controlled by the thermostatically controlled valve 17 which controls the flow of fuel gas from the fuel gas reservoir 14 to the combustion chamber 10. By appropriately setting the temperature of the thermostatically controlled valve 17, the temperature of the herbal matter 2 in the vaporising chamber 9 is maintained at the vaporising temperature of the constituent or constituents of the herbal matter to be vaporised.

Heat from the gas catalytic combustion element 12 is transferred by radiation and convection into the portion of the body member 7 which forms the combustion chamber 10, and in turn is conducted through the portion of the body member 7 which forms the socket element 25 of the vaporising chamber 9 into the herbal matter 2 in the vaporising chamber 9. Heat is also conducted through the heat conducting spigot 43 from the base wall 27 of the socket element 25 directly into the herbal matter 2, and heat is further transferred from the base wall 27 of the socket element 25 directly into the herbal matter 2 through the heat transfer spigots 47 of the heat transfer element 45.

As the herbal matter in the vaporising chamber 9 is brought up to the vaporising temperature of the vaporisable constituents to be vaporised, the vaporisable constituents of the herbal matter in the vaporising chamber 9 commence to vaporise. By drawing on the mouthpiece 38, air is drawn into the vaporising chamber 9 through the air passageways 42 between the side wall 26 of the socket element 25 and the side wall 34 of the plug element 30. Vapours evaporated from the vaporisable constituents of the herbal matter are entrained in the air as the air is being drawn through the vaporising chamber 9 to form the aerosol. The aerosol is in turn drawn through the mouthpiece 38 for inhaling thereof.

When the herbal matter 2 in the vaporising chamber 9 is spent, the plug element 30 is disengaged from the socket element 25, and the spent herbal matter 2 is removed from the hollow interior region 31 of the plug element 30. The heat transfer element 45 may then, if desired, be removed by hand from the hollow interior region 28 of the socket element 25 for cleaning of the heat transfer element 45 and the hollow interior region 28 of the socket element 25. On completion of cleaning, the heat transfer element 45 is replaced by hand in the socket element 25 by engaging the carrier ring 46 in the groove 50 in the base wall 27 of the socket element 25, and the vaporising device 1 is ready for the next charge of herbal matter.

It has been found that the provision of the heat transfer element 45 according to the invention in the vaporising chamber significantly increases the heat transfer efficiency of heat into the herbal matter. Firstly, it has been found that the heat transfer element 45 maintains the temperature of the herbal matter at a more constant and even temperature throughout the vaporising chamber than where the vaporising device is provided without the heat transfer element. Secondly, it has been found that the temperature of the herbal matter in the vaporising chamber is maintained substantially constant at the vaporising temperature of the vaporisable constituent or constituents more effectively and without any significant temperature transitions above and below the vaporising temperature of the vaporisable constituent or constituents of the herbal matter where the heat transfer element 45 is provided in the vaporising chamber, as opposed to where the heat transfer element is omitted from the vaporising chamber. Thirdly, it has been found that the heat transfer element 45 more quickly raises the temperature of the herbal matter in the vaporising chamber to the vaporising temperature of the vaporisable constituent or constituents from start-up of the vaporising device. Fourthly, by virtue of the fact that the heat transfer element is removeably engageable in the vaporising chamber, the heat transfer element may be readily easily removed by hand for cleaning thereof and for cleaning of the hollow interior region of the socket element of the vaporising chamber and of the heat conducting spigot 43 therein.

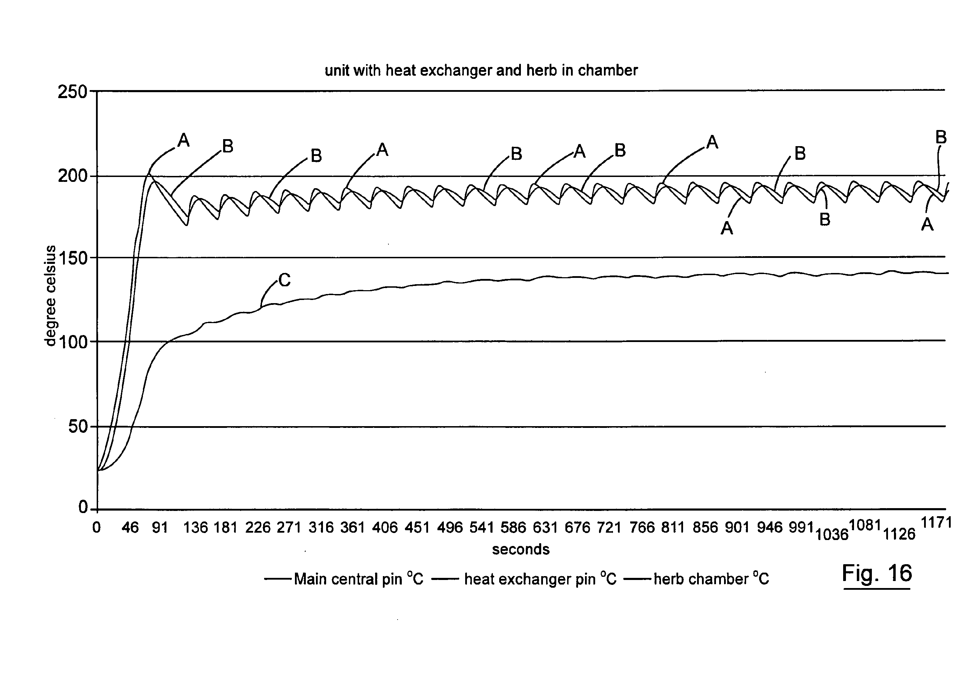

Fig. 16 illustrates three graphs A, B and C, which represent plots of temperature in degrees centigrade, which is plotted on the Y-axis, against time in seconds, which is plotted on the X-axis, of the vaporising device 1 during operation from start-up of the device 1. Graph A represents the temperature plotted against time of the heat conducting spigot 43. Graph B represents a plot of temperature against time of one of the heat transfer spigots 47 of the heat transfer element 45, while graph C represents the temperature plotted against time of an outer surface of the plug element 30 adjacent the side wall 26 of the socket element 25.

As can be seen from Fig. 16, the temperature of the heat conducting spigot 43 rises rapidly to approximately 200°C in just over one minute from start-up and then settles at a temperature of approximately 187°C varying between 185°C and 190°C. The temperature of the heat transfer spigot 47 of the heat transfer element 45 also rises rapidly to a temperature of approximately 195°C in approximately one and a quarter minutes from start-up. The temperature of the heat transfer spigot 47 then settles at a temperature of approximately 187°C, similar to that of the heat conducting spigot 43 varying between approximately 186°C and 188°C. The temperature of the heat conducting spigot 43 never falls below 165°C, and the temperature of the heat transfer spigot 47 never falls below 175°C. Thus, the heat conducting spigot 43 and the heat transfer spigots 47 are maintained within the vaporising temperature of the vaporisable constituent or constituents of the herbal matter, which lies within the range 165°C to 190°C in just over one minute from start-up.

The temperature of the outer surface of the side wall 34 of the plug member 30 represented by the graph C shows how the temperature of the outer surface of the side wall 34 only reaches a temperature of approximately 135°C to 140°C after approximately nine minutes. However, by virtue of the fact that the heat transfer spigots 47 of the heat transfer element 45 engage the herbal matter in the vaporising chamber 9 adjacent the side wall 34 of the plug element 30, the heat transfer spigots 47 being at a temperature of the order of 187°C maintain the herbal matter adjacent the side wall 34 of the plug element 30 well up to the vaporising temperature of the vaporisable constituent or constituents of the herbal matter.

Furthermore, the provision of the heat transfer spigots 47 of the heat transfer element 45 rapidly raise the temperature of the herbal matter adjacent the side wall 34 of the plug element 30 to the ignition temperature from start-up, and as discussed above, within approximately one and a quarter minutes from start-up of the vaporising device 1.

Thus, without the heat transfer spigots 47 of the heat transfer element 45, the temperature gradient from the heat conducting spigot 43 across the herbal matter to the side wall 34 of the plug element 30 would be such that herbal matter adjacent and relatively close to the side wall 34 of the plug element 30 might never be raised to a temperature sufficient to vaporise the vaporisable constituent or constituents in the herbal matter, and if the herbal matter adjacent the side wall 34 of the plug element 30 were raised to a sufficient temperature to vaporise the vaporisable constituent or constituents of the herbal matter, the herbal matter would not be raised to such a temperature for a considerable time after start-up of the vaporising device 1, and most likely not for approximately nine minutes from start-up.

Thus, the provision of the heat transfer element 45 with the heat transfer spigots 47 significantly enhances the efficiency of operation of the vaporising device, as well as reduces the time from start-up to raise substantially all the herbal matter in the vaporising chamber to its vaporisation temperature over and above vaporising devices known heretofore.

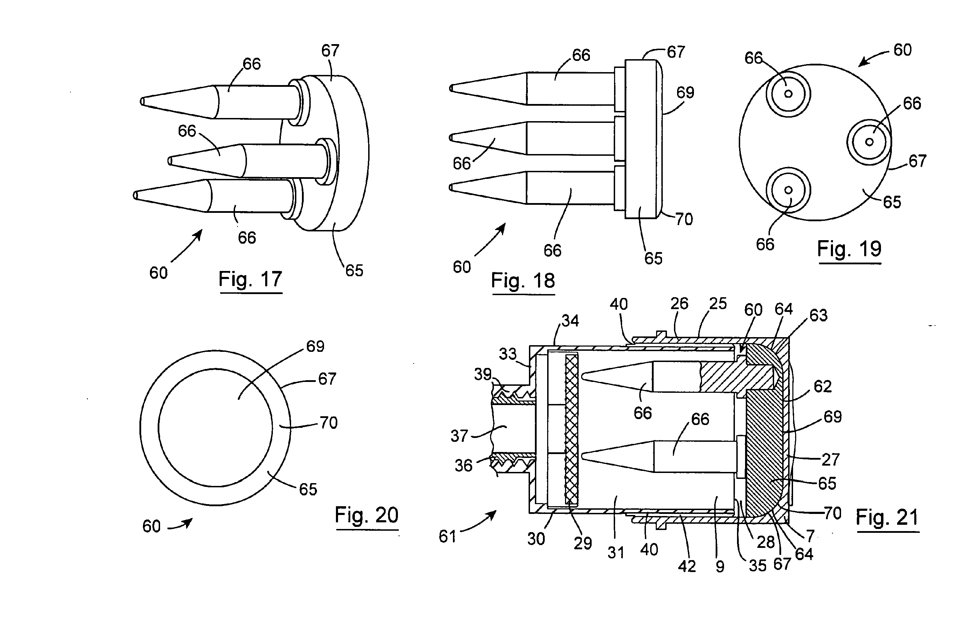

Referring now to Figs. 17 to 21, there is illustrated a heat transfer element according to another embodiment of the invention, indicated generally by the reference numeral 60, which is suitable for use in a vaporising device also according to the invention, only a portion of which is illustrated in Fig. 21 and is indicated generally by the reference numeral 61. The vaporising device 61 is substantially similar to the vaporising device 1, and similar components are identified by the same reference numerals. The only difference between the vaporising device 61 and the vaporising device 1 is that in the vaporising device 61 the heat conducting spigot is omitted from the vaporising chamber 9, and the base wall 27 defines a flat heat transfer surface 62 which terminates in a radiused peripheral heat transfer surface 63 adjacent the side wall 26, which is formed by a radiused fillet 64 extending around the base wall 27 and the side wall 26.

The heat transfer element 60 according to this embodiment of the invention comprises a carrier means provided by a carrier member in the form of a circular carrier plate 65. Three heat transfer spigots 66 which are similar to the heat transfer spigots 47 of the vaporising device 1 are rigidly secured to and are in heat transfer engagement with the carrier plate 65. The heat transfer spigots 66 are equi-spaced circumferentially around the carrier plate 65, and are spaced apart inwardly from a peripheral edge 67 of the carrier plate 65 so that the heat transfer spigots 66 extend into the hollow interior region 31 of the plug element 30 spaced apart from the side wall 34 of the plug element 30. The carrier plate 65 and the heat transfer spigots 66 are of similar material, which in this case may be brass, stainless steel or aluminium.

An under heat transfer surface 69 of the carrier plate 65 is flat and terminates in a radiused peripheral heat transfer surface 70 adjacent the peripheral edge 67. The radius of the radiused peripheral surface 70 of the carrier plate 65 is similar to the radius of the radiused fillet 64 between the base wall 27 and the side wall 26 of the socket element 25, and the diameter of the carrier plate 65 is substantially similar to the diameter of the base wall 27, so that the under surface 69 of the carrier plate 65 abuts the flat surface 62 of the base wall 27 with heat conducting engagement, and the radiused peripheral surface 70 of the carrier plate 65 abuts the radiused fillet 64 also with heat conducting engagement for heat transfer from the base wall 27 into the carrier plate 65 and in turn through the heat transfer spigots 66 to the herbal matter in the vaporising chamber 9. In this embodiment of the invention the heat transfer element 60 is removable and replaceable by hand in the vaporising chamber 9 for cleaning thereof and for permitting cleaning of the vaporising chamber 9.

Otherwise, the heat transfer element 60 and the vaporising device 61 are similar to the heat transfer element 45 and the vaporising device 1.

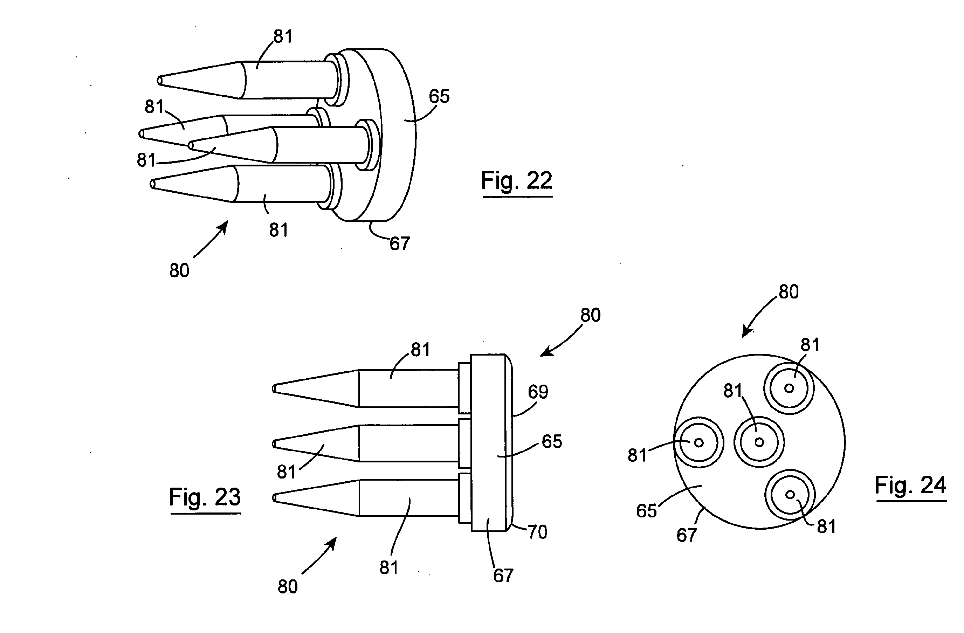

Referring now to Figs. 22 to 24, there is illustrated a heat transfer element 80 according to another embodiment of the invention for use in the vaporising device 61. The heat transfer element 80 is substantially similar to the heat transfer element 60 of Figs. 17 to 21 and similar components are identified by the same reference numerals. The only difference between the heat transfer element 80 and the heat transfer element 60 is that an additional heat transfer spigot 81 extends centrally from the carrier plate 65. Otherwise, the heat transfer element 80 and its use in the vaporising device 61 is similar to that described with reference to the heat transfer element 60.

While the vaporising device has been described as being gas powered, it will be appreciated that the vaporising device may be powered by any other suitable heating means, for example, an electrically powered heating means, such as electrical heating elements. Such heating elements may, for example, be embedded in the body member adjacent the base wall of the socket element or may be located in the fixed heat conducting spigot. Where the heating means comprises an electrically powered heating means, the heating means may comprise an electrically powered positive temperature control resistive heating element, a conventional resistive heating element, an induction heating element, a ceramics based heating element, or any other such electrically powered heating element.

While the vaporising chamber of the vaporising device has been described as being formed by a socket and plug element arrangement, any other suitable construction of vaporising chamber may be used. While it is desirable, it is not essential to provide the vaporising device with either a safety cut-out valve or a thermostatically controlled valve. However, where the vaporising device is provided without a thermostatically controlled valve, it is envisaged that the thermal mass of the body member 7 would be such as to maintain the temperature in the vaporising chamber 9 at substantially the vaporising temperature of the vaporisable constituent or constituents in the herbal or other vaporisable matter which are to be vaporised.

While the vaporising device according to the invention has been described as being suitable for vaporising vaporisable constituents in herbal matter to produce an aerosol, it is envisaged that the vaporising device may be used for vaporising any vaporisable constituent to produce an aerosol in any type of matter, whether herbal matter or otherwise. It is envisaged that the vaporising device may be used for vaporising medicinal compounds and the like, which would be placed in the vaporising chamber.

While the heat transfer element has been described as comprising a carrier means in the form of a carrier ring and a carrier plate, the carrier means may be of any other suitable construction and/or of any other suitable shape. Additionally, while specific numbers of heat transfer spigots have been described as extending from the carrier means, any other number of heat transfer spigots may be provided extending from the carrier ring. Indeed, in certain cases a single heat transfer spigot only may be provided. While the carrier ring and carrier plate have been described as being of a specific shape, and while the heat transfer spigots have been described as being of a specific shape, the carrier ring, the carrier plate and the heat transfer spigots may be of any other suitable shape and construction.

Additionally, it will be appreciated that provision may be made to more positively but releasably locate the carrier ring, carrier member or other suitable carrier means in the vaporising chamber, and it is envisaged in certain cases that the heat transfer element may be releasably secured in the vaporising chamber with a snap-fit action.

It is also envisaged that where the vaporising chamber is provided in the form of a socket element only, the heat transfer element would be located in the socket. Additionally, it is envisaged that the plug element of the vaporising chamber may be formed in the body member, and in which case, the heat transfer element would be located in the hollow interior region of the plug element.

It is also envisaged that the vaporising chamber and the combustion chamber may be formed in separate housings, and a suitable heat transfer means would be provided for transferring heat from the combustion chamber housing to the vaporising chamber housing.

While the vaporising devices and the components thereof and the heat transfer elements have been described as being of specific materials, it will be readily apparent to those skilled in the art that the vaporising devices and its components thereof as well as the heat transfer elements may be of any other suitable material.

## Claims

1. A vaporising device for vaporising a vaporisable constituent of vaporisable matter, the vaporising device (1,61) comprising a vaporising chamber (9) for the vaporisable matter, a heating means (12), a heat transfer means (45,47,60,66,80,81) extending into the vaporising chamber (9) adapted to contact the vaporisable matter therein for transferring heat to the vaporisable matter, **characterised in that** the heat transfer means (45,47,60,66,80,81) is adapted to be removeably engageable in the vaporising chamber (9).

2. A vaporising device as claimed in Claim 1 **characterised in that** the vaporising chamber (9) is formed within a body member (7), and the heating means (12) is adapted to heat the body member (7), the heat transfer means (12) being adapted to engage the body member (7) within the vaporising chamber (9) for transferring heat from the body member (7) to the vaporisable matter.

3. A vaporising device as claimed in Claim 2 **characterised in that** the heat transfer means (45,60,80) comprises a heat transfer element (45,60,80) comprising a carrier means (46,65), and at least one heat transfer spigot (47,66,81) extending from the carrier means (46,65), the carrier means (46,65) being adapted to engage the body member (7).

4. A vaporising device as claimed in Claim 3 **characterised in that** a plurality of spaced apart heat transfer spigots (47,66,81) extend from the carrier means (46,65).

5. A vaporising device as claimed in Claim 3 or 4 **characterised in that** the carrier means (46,65) defines a heat transfer surface (48,69,70) adapted to abut a corresponding heat transfer surface (51,62,64) of the body member (7) within the vaporising chamber (9) for heat transfer therebetween, the heat transfer surface (51,62,64) of the body member (7) defining the heat transfer surface (48,69,70) of the carrier means (46,65).

6. A vaporising device as claimed in Claim 5 **characterised in that** the carrier means (46,65) defines an opening (49) extending therethrough for accommodating a fixed heat conducting spigot (43) extending from the body member (7) into the vaporising chamber (9), the heat conducting spigot (43) being rigidly connected to the body member (7).

7. A vaporising device as claimed in Claim 5 or 6 **characterised in that** the carrier means (46,65) comprises one of a carrier plate member (65) and a carrier ring (46).

8. A vaporising device as claimed in any of Claims 5 to 7 **characterised in that** the body member (7) forms a socket element (25) having a base (27), and a side wall (26) extending from and around the base (27) and defining with the base (27) the vaporising chamber (9), and an annular groove (50) is formed extending around and into the base (27) of the socket element (25) for accommodating the carrier means (46,65) of the heat transfer element (45,60,80), and the heat transfer surface (51,62,64) of the body member (7) is formed by the surface (51) of the annular groove (50), and a hollow plug element (30) is releasably engageable in the socket element (25), the plug element (30) defining a hollow interior region (31) for the vaporisable matter and an open mouth (35) to the hollow interior region (31), the plug element (30) being engageable in the socket element (25) for defining with the socket element (25) the vaporising chamber (9), and the plug element (30) is adapted to engage the socket element (25) so that when the plug element (30) is engaged in the socket element (25), the heat transfer means (45,60,80) extends into the hollow interior region (31) of the plug element (30) to contact the vaporisable matter therein.

9. A vaporising device as claimed in Claim 8 **characterised in that** the plug element (30) co-operates with the socket element (25) to define an air accommodating passageway (42) into the vaporising chamber (9) between the plug element (30) and the socket element (25), the air accommodating passageway (42) defined between the plug element (30) and the socket element (25) being adapted to pre-heat air to the vaporising chamber (9), and the air accommodating passageway (42) being located relative to the vaporising chamber (9) so that air flows through the air accommodating passageway (42) in a direction opposite to the direction of flow of air through the vaporising chamber (9).

10. A vaporising device as claimed in Claim 8 or 9 **characterised in that** a communicating means (36) extends from the plug element (30) for accommodating an aerosol from the vaporising chamber (9), the communicating means (36) terminating in a mouthpiece (38).

11. A vaporising device as claimed in any of Claims 2 to 10 **characterised in that** the heating means (12) comprises one of a gas powered heating means and an electrically powered heating means.

12. A vaporising device as claimed in any preceding claim **characterised in that** the heat transfer means (45,60,80) is adapted to be manually removeable and insertable in the vaporising chamber (9).

13. A heat transfer element adapted for use in a vaporising device (1,61) of the type comprising a vaporising chamber (9) for vaporisable matter, the heat transfer element (45,60,80) being adapted to contact vaporisable matter in the vaporising chamber (9) for transferring heat to the vaporisable matter, **characterised in that** the heat transfer element (45,60,80) is adapted for removable engagement in the vaporising chamber (9).

14. A heat transfer element as claimed in Claim 13 **characterised in that** the heat transfer element (45,60,80) comprises a carrier means (46,65), and at least one heat transfer spigot (47,66,81) extending from the carrier means (46,65).

15. A heat transfer element as claimed in Claim 14 **characterised in that** the carrier means (46,65) defines a heat transfer surface (48,69,70) adapted to abut a corresponding heat transfer surface (51,62,64) of the body member (7) within which the vaporising chamber (9) is formed for heat transfer from the body member (7) to the carrier means (46,65).
